# EUROPÄISCHE PATENTSCHRIFT

(11) **EP 1 421 993 B1**
(45) Veröffentlichungstag und Bekanntmachung des Hinweises auf die Patenterteilung: **21.02.2007**
(21) Anmeldenummer: 03025228.2
(22) Anmeldetag: 05.11.2003
(51) Int. Cl.: B01L 3/00, B01D 39/08, B01D 39/20

(54) **Vorrichtung zur Separierung und Ausgabe von Plasma**
Apparatus for separating and delivering plasma
Dispositif de separation et livraison du plasma

(30) Priorität: 11.11.2002 DE 10252223
(43) Veröffentlichungstag der Anmeldung: 26.05.2004
(73) Patentinhaber: Roche Diagnostics GmbH, 68305 Mannheim (DE); F.HOFFMANN-LA ROCHE AG, 4070 Basel (CH)
(72) Erfinder: Frey, Guenter, 67158 Ellerstadt (DE); Ladiges, Norbert, Dr., 68782 Bruehl (DE); Noetzel, Siegfried, Dr., 69259 Wilhelmsfeld (DE); Roesicke, Bernd, 68305 Mannheim (DE)

(56) Entgegenhaltungen:
- DE-A- 19 629 656
- US-A- 4 477 575
- US-A- 5 130 231

## Beschreibung

Die Erfindung bezieht sich auf das Gebiet der Plasmagewinnung, die besonders bei analytischen Verfahren zur Bestimmung einer Konzentration von Blutbestandteilen eine Rolle spielt. Solche Bluttests können in vielen Fällen nicht mit Vollblut durchgeführt werden, da dieses korpuskuläre Bestandteile (Blutkörperchen) enthält, die bei der Ausführung des Verfahrens störende Einflüsse zur Folge hätten. Es ist deshalb notwendig, zur Durchführung vieler analytischer Verfahren zunächst das Plasma aus dem Vollblut zu gewinnen, das möglicht frei von zellulärem Material sein sollte.

Ein gebräuchliches Verfahren zur Gewinnung von Plasma für Bluttests ist das Zentrifugationsverfahren, in dem aufgrund von Zentrifugalkräfte zelluläre Bestandteile des Blutes abgetrennt werden. Dieses Verfahren ist aufwendig und eignet sich insbesondere dann nicht, wenn nur geringe Mengen Plasma für eine Analyse benötigt werden. Besonders bei modernen miniaturisierten Tests zeigt sich jedoch, dass Plasmamengen nur im Bereich von einigen Mikrolitern verwendet werden. Dies gilt insbesondere für die sogenannten trägergebundenen Tests, bei denen ein möglichst kleines und kompaktes Analysesystem, beispielsweise in Form eines Teststreifens, vorliegt. Hierbei sind alle für die Durchführung des Tests notwendigen Reagenzien und sonstige Mittel in den Teststreifen integriert. Zur Bestimmung eines Analyten wird die Probenflüssigkeit in Kontakt mit einem derartigen Analyseelement gebracht. Das im Testelement enthaltene Reagenz reagiert innerhalb eines kurzen Zeitraumes mit dem zu bestimmenden Analyten, so dass an dem Analyseelement eine physikalisch nachweisbare Veränderung erfolgt. Eine derartige Veränderung kann beispielsweise eine Farbänderung oder eine Änderung einer elektrischen Messgröße sein. Mit Hilfe eines Auswertegerätes wird diese Veränderung vermessen und berechnet, so dass ein analytisches Resultat ausgegeben werden kann.

Ein Beispiel für ein Analysesystem, das mittels eines derartigen Testelementes einen Analyten aus Plasma bestimmt, ist der HDL-Test (High Density Lipoproteins). Die Bestimmung der HDL-Konzentration im Blut ist unter anderem zur Risikoabschätzung einer koronaren Herzerkrankung von Bedeutung und dient damit zur Diagnose einer der häufigsten Erkrankungen in neuerer Zeit.

Die Ausprägung einer koronaren Herzerkrankung ist anhand einiger bekannter Parameter, wie z. B. das Gesamtcholesterin, im Blut, Plasma oder Serum zu erkennen. Da die Konzentration des Gesamtcholesterins für die individuelle Risikoabschätzung nur bedingt geeignet ist, werden in modernen Analyseverfahren die Lipoproteine niedriger Dichte (Low Density Lipoproteins = LDL) sowie Lipoproteine hoher Dichte (HDL) separat voneinander quantifiziert. Bei der Auswertung der Analyseverfahren muss dabei berücksichtigt werden, dass zwischen dem LDL-Cholesterin und einer koronaren Herzerkrankung eine positive, jedoch zwischen dem HDL-Cholesterin und der Erkrankung eine negative Korrelation besteht. Klinische Studien haben bewiesen, dass in erster Nährung die Bestimmung sowohl des HDLs als auch des Gesamtcholesterins für eine Risikoabschätzung ausreichend ist. In der diagnostischen Praxis wird zur Zeit vorzugsweise dieser Weg beschritten.

Die Bestimmung des HDL-Cholesterins erfolgt beispielsweise mittels eines Analyseelements, wie es im Stand der Technik bekannt ist (z. B. HDL-Testelemente der Firma Roche Diagnostics GmbH). Da hierbei eine separate Bestimmung des HDL-Cholesterins erfolgt, müssen die anderen vorhandenen Lipoproteinklassen von den restlichen Blutbestandteilen abgetrennt werden, sodass die Bestimmung des HDL-Cholesterins aus Plasma erfolgt. Ein solcher Test benötigt beispielsweise ein Plasmavolumen von circa 40 µl, damit eine Konzentrationsbestimmung unabhängig vom aufgegebenen Plasmavolumen durchgeführt werden kann. Zur Bestimmung des HDL-Cholesterins kann dabei nur reines Plasma verwendet werden, in dem weitestgehend keine Blutbestandteile enthalten sind. Zur Bestimmung der HDL-Konzentration wird weiterhin ein Komplexbildner verwendet, der ebenfalls in dem Testelement integriert ist. Die Plasmaaufgabe erfolgt nun auf den Bereich des Testelementes, in dem der Komplexbildner vorhanden ist. Zur Analyse des HDL-Cholesterins wird z. B. im Stand der Technik der Komplexbildner EDTA eingesetzt.

Eine Bestimmung eines Analyten aus reinem Plasma kann jedoch auch mittels eines Testelementes erfolgen, bei dem kein Komplexbildner zur Bestimmung eines Analyten notwendig ist. Derartige Testelemente werden beispielsweise zur Enzymbestimmung verwendet und sind im Stand der Technik unter anderem im Dokument DE 3130749 beschrieben. Testelemente, die einen Analyten aus reinem Plasma bestimmen, jedoch keinen Komplexbildner enthalten, sind häufig so beschaffen, dass eine Plasmaseparation durch das Testelement selbst geleistet werden kann. Hierfür beinhalten derartige Testelemente zusätzlich zu einer Reagenzschicht eine Separationsschicht. Zur Vermessung eines Analyten wird zunächst Vollblut auf die Separationsschicht aufgegeben. Innerhalb der Separationsschicht werden Blutbestandteile von Plasma getrennt, wobei das Plasma zu der Reagenzschicht weitergeleitet wird. Die Bestimmung eines Analyten kann somit aus reinem Plasma erfolgen, obwohl Blut auf das Testelement aufgegeben wurde. Eine derartige im Testelement integrierte Plasmaseparationsschicht, kann hingegen bei der Verwendung von Komplexbildnern nicht eingesetzt werden. Muss ein Komplexbildner zur Bestimmung eines Analyten verwendet werden, zeigt sich, dass eine Separation des Plasmas von Blut durch den Komplexbildner im Testelement verhindert wird. Eine Plasmaseparation kann folglich dann nicht mehr durch das Test-element geleistet werden, wenn das Testelement einen Komplexbildner beinhaltet.

Da zur Bestimmung des HDL-Cholesterins mittels eines oben beschriebenen Testelementes einerseits ein Komplexbildner notwendig ist, und andererseits eine Trennung des Plasmas vom Blut erforderlich ist, ergibt sich hieraus zwingend, dass eine Plasmaabtrennung vom Blut im µl-Maßstab bereits vor Blutaufgabe auf das Testelement geleistet werden muss.

Die Bestimmung des HDL-Cholesterins ist hierbei nur ein wichtiges Beispiel für eine Analytbestimmung, die geringe Mengen reines Plasma zur Analyse benötigen. Weitere Anwendungsgebiete für die Verwendung von freigesetztem Plasma zeigen sich im Bereich der klinischen Analyse. Da derzeitig in der diagnostischen Praxis vorzugsweise Teststreifen als Analysesysteme verwendet werden, steigt das Bedürfnis, einfache Verfahren zur Gewinnung geringer Mengen an Plasma verwenden zu können, so dass insgesamt eine Vereinfachung sowie ein schneller Ablauf der Analyseverfahren resultiert.

Im Stand der Technik werden diesbezüglich mehrere Verfahren beschrieben, die die Gewinnung von geringen Mengen Plasma vereinfachen sollen. Dabei soll aus ebenfalls kleinen Blutvolumina das Plasma gewonnen werden, damit dem Patienten eine aufwendige Blutentnahme, wie sie z. B. für ein Zentrifugationsverfahren notwendig wäre, erspart bleibt.

Seit vielen Jahren werden Filtrationsverfahren diskutiert und teilweise mit Erfolg verwendet, bei denen unterschiedliche Filtermedien, insbesondere Membran- und Glasfilter, zum Einsatz kommen. Frühere Beispiele für eine Filtrationstechnik sind in US-Patentschriften US 3,791,933 und US 4,477,575 beschrieben. Ein neueres Beispiel mit einer aufwendigen Kombination aus Membran- und Glasfiltern wird im Patent US 5,922,210 dargestellt. Mit Hilfe eines Mikrobauteils werden kleine Plasmamengen durch Mikrofiltration gewonnen. Die Abtrennung der Blutkörperchen erfolgt mit einem sogenannten Sperrkanal, der so klein ist, dass Blutkörpercheri nicht durch den Sperrkanal durchfließen können. Die Herstellung einer Vorrichtung mit einem derartigen Kanal erfordert jedoch spezielle Herstellungsverfahren, die aufwendig sind. Die genannten Plasmagewinnungsmethoden haben weiterhin den Nachteil, dass ein hohes Risiko besteht, die feinen Poren durch mechanischen Verschluss oder durch Addition von zellulärem Material an den Porenwänden zu verstopfen. Dadurch wird die Filterkapazität begrenzt. Eine Vergrößerung der Filterkapazität würde jedoch einen größeren Raumbedarf des Filtermediums bedingen. Dies würde wiederum die Relation zu dem aufgebrachten Probenvolumen und gewonnenen Plasmavolumen ungünstig beeinflussen.

Im Dokument US 4,477,575 wird ein Filterungsprozess zur Plasmagewinnung beschrieben mit einer Glasfaserschicht, durch den die Relation zwischen Probenvolumen und gewonnenen Plasmavolumen verbessert wird. Das Volumen des abzutrennenden Plasmas beträgt hierfür vorzugsweise weniger als 30 % vom Saugvolumen der Glasfaserschicht. Der Filterungsprozess erfolgt, nach dem Blut auf die Glasfaserschicht aufgegeben wurde und wird ausschließlich aufgrund von Schwerkraft angetrieben, sodass die Gewinnung des Plasmas entsprechend zeitaufwendig ist.

Um die Plasmagewinnung zu beschleunigen, werden im Stand der Technik weitere Möglichkeiten zur Plasmagewinnung beschrieben. In der Patentanmeldung EP 747105 wird zunächst in einem Gefäß ebenfalls eine Glasfaser gelagert, auf die das Blut aufgegeben wird. Mittels eines Stempels wird Druck auf die Glasfaser und das darin enthaltene Blut ausgeübt, sodass der Filterungsprozess beschleunigt wird. Das Blut wird somit durch die Glasfaser gepresst, wobei sich das Plasma von den restlichen Blutbestandteilen trennt. Das Plasma wird über einen Auslass ausgegeben. Nachteil der beschriebenen Vorrichtung ist jedoch, dass aufgrund des Filtervorganges größere Probenmengen Blut benötigt werden. Des Weiteren führt das Ausdrücken der Glasfaser zur Zerstörung der korpuskulären Blutbestandteile, sodass kein reines Plasma gewonnen werden kann.

Auf einem ähnlichen Prinzip basiert ein Gefäß zur Plasmagewinnung, das in der Patentanmeldung EP 0 785 012 beschrieben wird. Hierbei wird ebenfalls Druck auf ein Filtermaterial ausgeübt, auf dem zuvor Blut aufgegeben wurde, sodass eine Plasmaseparation stattfindet. Wie bereits oben beschrieben, erfolgt auch hier aufgrund des Auspressvorgangs eine Zerstörung der Blutkörperchen, sodass keine reine Plasmagewinnung stattfindet. Wird das Plasma durch die Zerstörung der Blutkörperchen erst einmal verunreinigt, ist es für die Verwendung einer Vielzahl von Analysetests nicht geeignet.

Aufgabe der Erfindung ist es, eine Vorrichtung sowie ein Verfahren bereitzustellen, das zur Gewinnung von möglichst reinem Plasma im Mikroliter-Maßstab aus Vollblut geeignet ist. Dabei sollen die beschriebenen Nachteile des Standes der Technik überwunden werden.

Die Erfindung beinhaltet eine Vorrichtung zur Separierung und Ausgabe von Plasma. Die Vorrichtung umfasst ein Trennelement, das ein Trennvlies als einen ersten Bereich beinhaltet, auf dem eine Blutaufgabe erfolgt. Im ersten Bereich des Trennelementes werden korpuskuläre Blutbestandteile im wesentlichen vollständig zurückgehalten, während vorteilhafterweise durch Kapillarkräfte des Trennelementes Plasma in ein Transportvlies als einen zweiten Bereich des Trennelementes geleitet wird. Das Trennelement ist dabei so in der Vorrichtung angeordnet, dass der erste Bereich des Trennelementes für den Benutzer zur Blutaufgabe zugänglich ist. Weiterhin beinhaltet die Vorrichtung eine Ausgabeeinheit, die nach der Plasmaseparierung auf den zweiten Bereich des Trennelementes wirkt, ohne dass auf den ersten Bereich des Trennelementes eine Einwirkung stattfindet, die z. B. zu einer Hämolyse des Blutes führen würde. Wirkt die Ausgabeeinheit ausschließlich auf den zweiten Bereich des Trennelementes, wird das separierte Plasma aus dem zweiten Bereich freigesetzt und über einen Auslass der Vorrichtung abgegeben.

Weiterhin beinhaltet die Erfindung ein System zum Nachweis von Analyten im Blut. Das System beinhaltet neben der erfindungsgemäßen Vorrichtung - wie beschrieben - weiterhin ein Testelement, das bei Aufgabe des durch die Vorrichtung separierten Plasmas den Nachweis eines Analyten im Plasma ermöglicht.

Die erfindungsgemäße Vorrichtung gewährleistet eine effektive Plasmagewinnung im Mikroliter-Maßstab bereits schon aus geringem Probenvolumina. Beispielsweise können aus 100 µl Blut Plasmavolumina von 30 µl oder mehr gewonnen werden. Die erfindungsgemäße Vorrichtung ist damit besonders geeignet im Bereich der modernen Analyse eingesetzt zu werden, da bereits bei der Entnahme von geringen Probenvolumina eine schnelle Plasmaseparation, sowie die Ausgabe des Plasmas vorteilhafterweise auf einen Teststreifen ermöglicht wird. Beträgt das aufgegebene Blutvolumen vorzugsweise 30 bis 150 µl, kann mittels der erfindungsgemäßen Vorrichtung hinreichend Plasmamengen gewonnen werden, die für handelsübliche Testelemente vorgeschrieben sind, damit eine Analytkonzentration unabhängig vom aufgegebenen Probenvolumen bestimmt werden kann. Mittels der erfindungsgemäßen Vorrichtung ist es folglich möglich, trotz geringer Blutvolumina hinreichend große Plasmamengen zu gewinnen, um den Anforderungen handelsüblicher Analyseverfahren insbesondere mit Testelementen zu genügen.

Die erfindungsgemäße Vorrichtung erlaubt weiterhin eine besonders einfache und kostengünstige Herstellung des Systems, da z. B. keine Mikrostrukturen (Mikrokanäle) beim Herstellungsprozess in die Vorrichtung integriert werden müssen. Die Separation des Plasmas erfolgt mittels eines Trennelementes, das vorteilhafterweise zur einmaligen Verwendung ausgelegt ist. Eine Verstopfungen der mikroporöser Strukturen bei mehrmaliger Verwendung sowie Verunreinigungen können somit vermieden werden.

Weitere wesentliche Vorteile der Erfindung ergeben sich durch die Freisetzung des Plasmas und das Abtrennen des Plasmas aus Blut, die im Sinne der Erfindung als zwei separate, hintereinanderfolgende Vorgänge durchgeführt werden. Somit ist es möglich, die Plasmagewinnung zu beschleunigen, ohne dass hierbei, wie im Stand der Technik üblich, Druck während des Separationsvorgangs auf die Probe ausgeübt wird. Erfindungsgemäß wird im wesentlichen nur durch Einwirkung auf den zweiten Bereich des Trennelementes das Plasma freigesetzt, wobei dieser Vorgang beliebig z. B. durch Überdruck, Unterdruck oder Eluierungsprozesse etc. beschleunigt werden kann. Von diesem Vorgang im wesentlichen unberücksichtigt bleibt der Schritt der Plasmaseparation, der unabhängig von dem Freisetzungsprozess durchgeführt wird und z. B. durch Kapillarkräfte, die innerhalb des ersten Bereiches des Trennelementes wirken, ebenfalls beschleunigt werden kann. Hierbei sollte jedoch darauf geachtet werden, dass eine Beschleunigung der Plasmaseparation nur so weit erfolgt, dass weiterhin eine zuverlässige Separation des Plasmas von den restlichen Blutbestandteilen sicher gewährleistet ist. Insbesondere sind hierbei Vorgänge, die z. B. Scherkräfte bedingen zu vermeiden, die eine Hämolyse während der Plasmaseparation bewirken würden. Die Erfindung ermöglicht folglich ein beschleunigtes Verfahren der Plasmaseparation, ohne das eine Kontamination des Plasmas mit restlichen Blutbestandteilen in Kauf genommen werden muss.

Im Bereich der modernen Analyse ist die Erfindung insbesondere zur Aufgabe von reinem Plasma auf Testelemente geeignet, die - wie beschrieben - einen Komplexbildner enthalten und aufgrund des Komplexbildners eine Plasmaseparation innerhalb des Testelementes selbst nicht verwirklicht werden kann.

Es ist jedoch auch eine Anwendungsmöglichkeit für Testelemente denkbar, die keinen Komplexbildner enthalten. Obwohl das Plasma hier handelsüblich auch mittels einer Separationsschicht im Testelement selbst abgetrennt werden kann und der Benutzer folglich bei dem Gebrauch dieser Testelemente nicht auf eine gesonderte Plasmaabtrennung angewiesen ist, erlaubt das erfindungsgemäße System z.B. hier einen vereinfachten Aufbau der Testelemente. Die Testelemente müssen somit nur noch über eine Reagenzschicht und nicht mehr über eine Separationsschicht bzw. Trennvlies verfügen. Hierdurch ergibt sich u. a. eine Reduktion der Produktionsschritte, die zur Kostenreduktion der Testelemente führen.

In Anlehnung an die im Stand der Technik beschriebenen Testelemente mit einer Separationsschicht bzw. Trennvlies ist eine bevorzugte Ausführungsform eines erfindungsgemäßen Trennelementes in einer ersten Nährung nach einem analogen Prinzip aufgebaut. Zur näheren Beschreibung handelsüblicher Testelemente mit Separationsschicht wird z. B. auf das Dokument DE 3130749 verwiesen. Das Dokument beschreibt ein Testelement, bei dem zunächst das Plasma vom Blut abgetrennt wird, sodass ausschließlich Plasma zu einer Reagenzschicht weitergeleitet wird. Mit Hilfe des Reagenzes erfolgt nun eine Bestimmung eines Analyten in Plasma. Zu diesem Zweck hat das Testelement eine auf dem Basisstreifen angeordnete, flache Separationsschicht, auf die an einem Ende die Blutprobe aufgegeben wird. Die Separationsschicht besteht aus Glasfasermaterial, das Blutkörperchen in der Nähe der Aufgabenstelle zurückhält. Das Blut-plasma breitet sich dagegen in der Schicht aus, sodass sich in dem von der Aufgabenstelle entfernten Bereich der Separationsschicht ein "Plasmasee" zur Verfügung steht. Üblicherweise befindet sich eine Reagenzschicht ober- oder unterhalb des Plasmasees, durch die anschließend eine Analytbestimmung im Plasma erfolgen kann. Zur Auswertung eines derartigen Testträgers werden entsprechende Auswertegeräte, wie sie im Stand der Technik bekannt sind, verwendet.

Nachteil dieser Analysesysteme ist jedoch, dass die Anwendung des so gewonnenen "Plasmasees" auf das beschriebene System beschränkt ist. Eine Analyse des im Plasma enthaltenen Analyten ist somit nur im Rahmen des Testträgersystems möglich, da ein Freisetzen des Plasmas aus dem Testträger nicht möglich ist.

Enthält eine erfindungsgemäße Vorrichtung ein Trennelement, das den Aufbau eines derartigen Testelementes vereinfacht wiedergibt, beinhaltet ein solches Trennelement beispielsweise in seinem ersten Bereich eine Separationsschicht, die im Folgenden auch als Trennvlies bezeichnet wird sowie in einem zweiten Bereich ein Transportvlies, in dem sich das Plasma in einem entfernten Bereich von der Separationsschicht sammelt. Eine bevorzugte Ausführungsform des Trennelementes hat folglich einen testträgeranalogen, streifenförmigen Aufbau, ohne dass eine Reagenzschicht vorhanden ist. Das Trennelement beinhaltet vorzugsweise in seinem Trennvlies einen Filter, der z. B. aus Glasfasermaterial besteht, sodass eine im wesentlichen vollständige Separation des Plasmas vom Blut gewährleistet wird. Weitere handelsübliche Vliese sind z. B. in dem Dokument EP 0 045 476 beschrieben oder im Handel unter dem Namen Whatman-Vlies erhältlich. Der Filterungsprozess wird hierbei erfindungsgemäß im wesentlichen nicht durch Druck unterstützt, sodass eine Zerstörung der Blutkörperchen im ersten Bereich des Trennelementes vermieden wird. Wirkt dennoch zur Unterstützung des Filterungsprozesses z. B. ein Unterdruck auf den ersten Bereich des Trennelementes, so ist darauf zu achten, dass ein Druck nur in dem Maße ausgeübt wird, dass hierdurch keine Hämolyse bewirkt wird.

Das Freisetzen des Plasmas erfolgt nun anschließend durch Einwirkung auf den zweiten Bereich des Trennelementes, ohne dass der erste Bereich des Trennelements beeinflusst wird. Eine Verunreinigung des Plasmas durch den Verfahrensschritt der Plasmafreisetzung wird somit erfindungsgemäß verhindert.

Vorzugsweise wird zur Freisetzung des Plasmas Druck auf den zweiten Bereich des Trennelementes ausgeübt, sodass das Plasma aus dem zweiten Bereich des Trennelementes ausgepresst wird. Wird Plasma aus dem Trennelement ausgepresst, ist hierbei zu beachten, dass ausschließlich auf den zweiten Bereich des Trennelementes Druck ausgeübt wird, in dem keine Blutbestandteile mehr enthalten sind, sodass eine Hämolyse vermieden wird. Prinzipiell sind vielfältige Möglichkeiten zur Freisetzung des Plasmas denkbar, wobei der Verfahrensschritt der Plasmafreisetzung erfindungsgemäß unabhängig von der Plasmaseparation stattfindet, sodass sich aus dem Verfahrensschritt der Plasmaseparation keine Einschränkungen für die Plasmafreisetzung ergeben. Ein weiteres Beispiel für die Plasmafreisetzung ist das Eluieren des separierten Plasmas. Über einen Auslass der Vorrichtung wird anschließend das freigesetzte Plasma dosiert abgegeben.

Für die Freisetzung des Plasmas erweist es sich weiterhin als vorteilhaft, wenn die Kräfte zur Freisetzung des Plasmas im wesentlichen senkrecht zur Ebene, in der sich das Trennelement befindet, auf den zweiten Bereich des Trennelementes wirken. Auf diese Weise kann gewährleistet werden, dass keine Beeinflussung des ersten Bereiches des Trennelementes erfolgt, die eine Kontamination des freizusetzenden Plasmas mit den restlichen Blutbestandteilen bedingen würde.

Hierbei ist z. B. eine Ausführungsform mit einem Stempel denkbar, bei der der Stempel innerhalb der Vorrichtung ober- oder unterhalb der Ebene, in der der zweite Bereich des Trennelementes positioniert ist, angeordnet ist. Durch ein Andrücken des Stempels gegen das Trennelement, wird somit ausschließlich Druck auf den zweiten Bereich des Trennelementes ausgeübt und das Plasma freigegeben.

Es ist weiterhin auch denkbar, dass zur sicheren Freisetzung von reinem Plasma zunächst der zweite Bereich des Trennelementes vom ersten Bereich des Trennelementes abgetrennt wird, so dass z. B. eine räumliche Trennung der Trennelementbereiche stattfinden kann. Hierbei wird vorteilhafterweise darauf geachtet, dass die Abtrennung des zweiten Bereiches vom ersten Bereich erst an einer Stelle des Trennelementes erfolgt, in dem im wesentlichen nur noch Plasma vorhanden ist, sodass keine korpuskulären Blutbestandteile im zweiten Bereich des Trennelementes das Plasma verunreinigen. Eine Freisetzung des Plasmas kann nun aufgrund der räumlichen Trennung auf vielfältige Weise durchgeführt werden, ohne dass eine Beeinträchtigung auf den ersten Bereich befürchtet werden muss.

Eine Abtrennung des zweiten Bereichs des Trennelementes von dem ersten Bereich kann z. B. durch eine Halterung verwirklicht werden, die mit dem zweiten Bereich des Trennelementes verbunden ist. Wird durch den Benutzer eine Kraft (Ziehen, Drücken, Drehen etc.) auf diese Halterung ausgeübt, wird diese Kraft direkt oder indirekt auf den zweiten Bereich des Trennelementes übertragen und führt hierdurch zur Abtrennung des zweiten Bereichs. Hierbei ist es z. B. möglich, dass durch eine Drehung der Halterung um vorzugsweise 90° ein Abtrennen des zweiten Bereiches vom ersten Bereich, der ortsfest innerhalb der Vorrichtung fixiert ist, bewirkt wird.

In einer weiteren vorteilhaften Ausführungsform erfolgt das Abtrennen des Trennelementes sowie das anschließende Freisetzen des Plasmas aus dem zweiten Bereich in zwei hintereinanderfolgenden Schritte, die durch Betätigung ein und derselbe Auslöseeinheit an der Vorrichtung erfolgt. Eine derartige Auslöseeinheit ist dann mit der Halterung der Vorrichtung in der Weise verbunden, dass bei einer ersten Betätigung der Auslöseeinheit zunächst eine Durchtrennung des Elementes bewirkt wird und eine weitere Betätigung der Auslöseeinheit zur Plasmafreisetzung führt. Auslöseeinheiten, wie beschrieben, sind vorteilhafterweise in Form eines Auslöseknopfes verwirklicht, der in einem ersten Schritt z. B. zunächst ein Drehen einer Halterung bewirkt, die mit dem zweiten Bereich des Trennelementes verbunden ist, sodass eine Drehung dieses Trennelementbereiches resultiert. Während der Drehbewegung des in der Halterung befestigten zweiten Bereiches verbleibt der erste Teil des Trennelementes ortsfest in der Vorrichtung. Die durch die Drehbewegung wirkenden Kräfte führen zur Teilung des Trennelementes. Hierbei ist es z. B. denkbar, dass ein Schneideelement in der Vorrichtung so positioniert ist, dass der zweite Bereich des Trennelementes während der Drehbewegung gegen das Schneideelement gedrückt wird. Eine Teilung des Trennelementes wird somit erleichtert und kann präzise durchgeführt werden. Wird auf ein Schneideelement verzichtet, kann ein Durchtrennen des Trennelementes ebenfalls durch ein Abreißen des ersten Bereiches vom zweiten Bereich bewirkt werden. Anschließend wird das Plasma mittels der Ausgabeeinheit freigesetzt.

Ist in einer vorteilhaften Ausführungsform der Vorrichtung das Trennelement als Einmalartikel ausgelegt, sodass eine irreversible Durchtrennung des Elementes nicht nachteilig ist, ist es weiterhin denkbar, die Halterung ebenfalls als fest mit dem Trennelement verbundenen Einmalartikel anzubieten. Für den Benutzer würde somit das Handling der Vorrichtung beim Wiedereinlegen eines neuen Trennelementes stark vereinfacht, da insbesondere bei älteren Personen eine Handhabung kleiner Gerätebauteile Schwierigkeiten hervorruft. Zur weiteren Vereinfachung der Handhabungsschritte bietet sich darüber hinaus eine Magazinierung der Halterung mit Trennelement in einem Spender an, der eine Ausgabe des Einmalartikels stückweise zulässt.

Weiterhin ist Gegenstand der Erfindung ein Verfahren zur Plasmaseparierung und der Ausgabe. Das Verfahren beinhaltet die Aufgabe von Blut auf einen ersten Bereich eines Trennelementes, welches einen ersten und einen zweiten Bereich beinhaltet. Mittels des Trennelementes wird das Plasma von anderen Blutbestandteilen separiert, wobei das Plasma in den zweiten Bereich des Trennelementes weitergeleitet wird und die verbleibenden Blutbestandteile im wesentlichen im ersten Bereich des Trennelementes zurückgehalten werden. Anschließend wird der zweite Bereich des Trennelementes in der Weise bearbeitet, dass das Plasma aus dem zweiten Bereich des Trennelementes freigesetzt wird. Hierbei ist darauf zu achten, dass keine Bearbeitung des ersten Bereiches des Trennelementes erfolgt, die beispielsweise durch eine Hämolyse eine Kontamination des Plasmas mit den zuvor abgetrennten Blutbestandteilen verursachen würde. Über einen Auslass der Vorrichtung erfolgt eine Ausgabe des freigesetzten Plasmas.

Vorteilhafte Ausführungsform des Verfahrens ergeben sich wie beschrieben. Vorzugsweise erfolgt das Verfahren zur Plasmaabtrennung und Freisetzung mittels einer Vorrichtung wie beschrieben.

Die erfindungsgemäße Vorrichtung sowie das erfindungsgemäße Verfahrens erlauben folglich eine einfach und schnell durchführbare Separierung von Plasma aus Blut im Mikroliter-Maßstab. Die Vorrichtung ist hierbei bequem zu handhaben und erlaubt eine preiswerte Herstellung. Beispielhaft werden nachfolgend einige vorteilhafte Ausführungsformen in den Figuren dargestellt und beschrieben.
- Figur 1:: Aufbau eines Trennelementes
- Figur 2:: Vorrichtung zur Separierung von Plasma
- Figur 3:: Vorrichtung zur Separierung von Plasma mit einer drehbar gelagerten Halterung

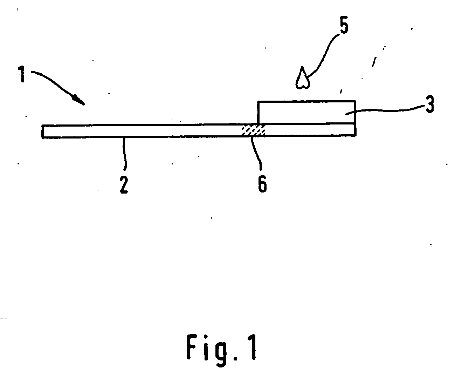

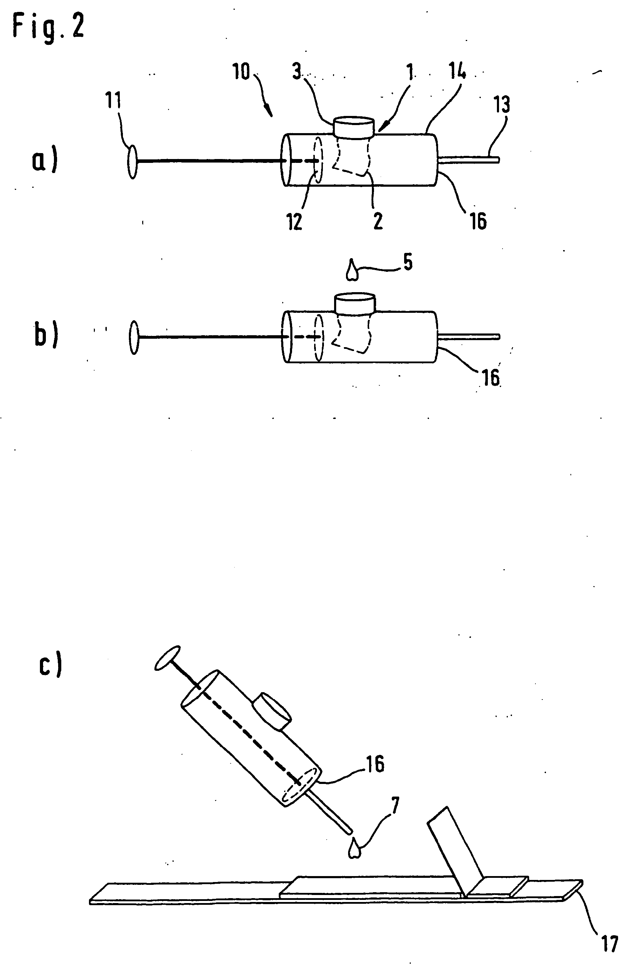

Figur 1 zeigt beispielhaft den Aufbau eines Trennelementes (1). Das Trennelement (1) enthält ein Transportvlies (2), das z. B. aus Glasfasern besteht. Auf dem Transportvlies (2) ist ein Trennvlies (3) angebracht, das aus einem Filtermedium besteht. Transport- und Trennvlies unterscheiden sich im wesentlichen anhand unterschiedlicher Dichte. Hierbei sind im Stand der Technik z. B. eine Dichte von 77 g/cm² für ein Trennvlies und 53 g/cm² für ein Transportvlies (Whatman-Vlies) angegeben. Die geringere Dichte des Transportvlieses ermöglicht einen schnellen Transport der Probe entlang des Vlieses, während die höhere Dichte des Trennvlieses eine sichere Separation des Plasmas von Blut gewährleistet. Bei Aufgabe eines Bluttropfens (5) gelangt das Blut in das Trennvlies (3). Aufgrund des Filtermediums im Trennvlies werden die Blutbestandteile vom Plasma getrennt und im Trennvlies (3) zurückgehalten. Das Plasma wird mittels Kapillarkräfte, die innerhalb des Transportvlieses wirken, weitergeleitet. Hierbei zeigt sich häufig, dass Blutbestandteile in geringer Konzentration durch Kapillarkräfte aus dem Trennvlies (3) in einen kleinen Bereich des Transportvlieses (2) gelangen können. Dieser Bereich wird als Übergangszone (6) bezeichnet und enthält kein reines Plasma. In einer vorteilhaften Ausführungsform der erfindungsgemäßen Vorrichtung wirkt deshalb bei der Freisetzung des Plasmas die Ausgabeeinheit nicht auf die Übergangszone des Transportvlieses, um eine Kontamination des übrigen Plasmas mit den hier vorhandenen Verunreinigungen zu vermeiden. Wie in Figur 2 gezeigt, wird das Aussparen der Übergangszone z. B. durch eine Abtrennung des zweiten Bereiches des Testelementes vom ersten Bereich jenseits dieser Übergangszone verwirklicht, sodass die Gewinnung von reinem Plasma gewährleistet ist.

Figur 2 a) bis c) zeigt beispielhaft ein Verfahren zur Plasmaseparierung mit einer erfindungsgemäßen Vorrichtung (10). Die Vorrichtung beinhaltet einen Hohlkörper (14), der über einen Auslass (13) verfügt. Innerhalb des Hohlkörpers ist das Trennelement (1) in der Weise angeordnet, dass das Trennvlies (3) aus dem Hohlkörper (14) ragt und für den Benutzer leicht zugänglich ist. Das Transportvlies (2) befindet sich innerhalb des Hohlkörpers (14). Die Vorrichtung beinhaltet weiterhin einen Stempel (12), der beweglich innerhalb der Vorrichtung (14) gelagert ist. Dabei ist der Radius des Stempels (12) im wesentlichen gleich dem inneren Radius des Hohlkörpers (14), sodass der Stempel (12) mittels eines Knopfes (11) innerhalb der Vorrichtung bewegt werden kann. Figur 2 b) zeigt eine Blutaufgabe (5) auf ein Trennvlies (3) eines Trennelementes (1). Gelangt das Blut in das Trennvlies, wird das Plasma entlang des Trennvlieses weitergeleitet, während die restlichen Blutbestandteile im Trennvlies zurückgehalten werden. Nach ca. 2 bis 10 Sekunden hat eine vollständige Plasmaseparierung stattgefunden. Das separierte Plasma wird nun in das Transportvlies (2) weitergeleitet. Durch Betätigung des Knopfes (11) wird zunächst der Stempel (12) gegen das Transportvlies (2) gedrückt, so dass dieser Bereich des Trennelementes innerhalb des Hohlkörpers (14) vom Stempel mitgerissen wird. Da das Trennvlies (3) im Hohlkörper ortsfest positioniert ist, kommt es zur Abtrennung des Transportvlieses vom Trennvlies, wobei eine Trennung jenseits der in Figur 1 dargestellten Übergangszone (6) erfolgt. Durch weitere Betätigung des Knopfes (11) wird das abgetrennte Transportvlies (2) gegen die Wand (16) des Gehäuses (14) gedrückt. Der Stempel (12) presst dabei das Plasma aus dem Transportvlies (2) aus und gibt diese frei. Über dem Auslass (13) der Vorrichtung erfolgt anschließend eine Plasmaabgabe (7). Das Plasma kann so z. B. auf ein Testelement (17) zur Bestimmung der HDL-Konzentration aufgetragen werden.

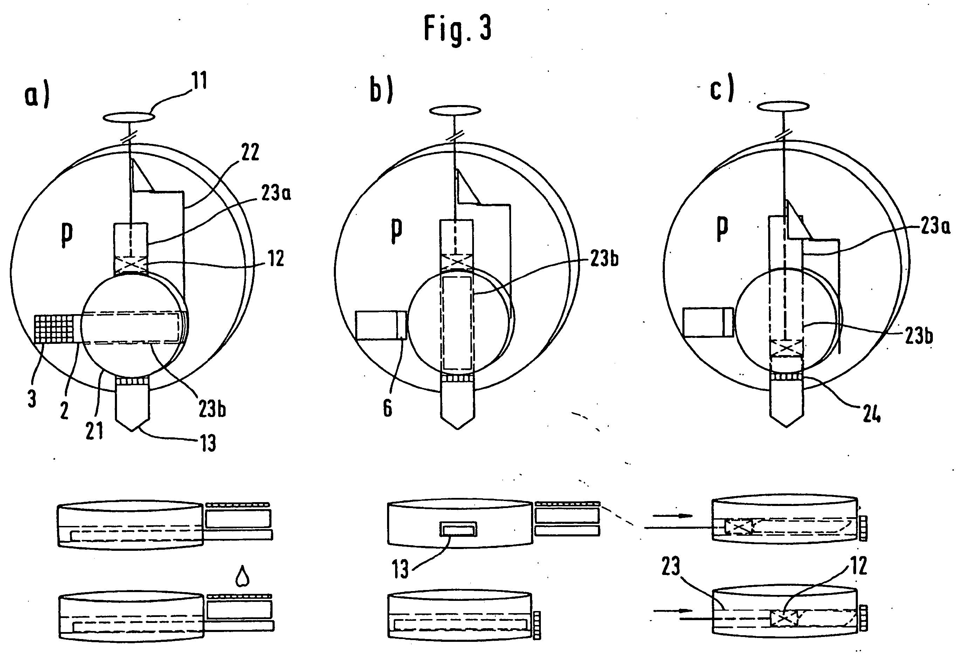

Figur 3 zeigt verschiedene Ansichten von einer weiteren Ausführungsart der Vorrichtung (a - c). Die Vorrichtung beinhaltet zusätzlich zu der in Figur 2 gezeigten Ausführungsform eine drehbar gelagerte Halterung (21), in der ein Trennelement (1) innerhalb eines Kanales (23) positioniert ist. Das Trennelement (1) ist dabei so in der Halterung positioniert, dass das Trennvlies (3) außerhalb der Halterung sowie der Vorrichtung ragt, so dass es - wie es in den Seitenansichten jeweils verdeutlicht wird - für den Benutzer zur Blutaufgabe leicht zugänglich ist. Die Vorrichtung verfügt weiterhin über einen Stempel (12), der mit dem Knopf (11) in Verbindung steht. Weiterhin kann mittels des Knopfes (11) ein Drehelement (22) betätigt werden. Zunächst erfolgt eine Blutaufgabe auf das Trennvlies (3) des Trennelementes (1), wie es in Figur 3 a) der Seitenansicht dargestellt ist. Nachdem die Separation des Plasmas vom Blut erfolgt ist, wird durch ein erstes Drücken des Knopfes (11) das Drehelement (22) betätigt. Durch eine nach unten gerichtete Bewegung des Drehelementes (22) wird die Halterung (21) um ca. 90° gedreht. Hierbei wird das Trennvlies (3) vom Transportvlies (2) abgetrennt, wobei die Übergangszone (6) des Transportvlieses (2) am Trennvlies (3) verbleibt. Durch weiteres Betätigen des Knopfes (11) wird nun der Stempel (12), der sich zunächst innerhalb des Kanales (23 a) befindet, in den Kanalbereich (23 b) überführt.

Hierdurch erfolgt ein Zusammenpressen des Transportvlieses gegen ein im Auslass (13) befindliches Sieb (24). Das Sieb (24) verfügt vorzugsweise über eine geringe Dicke von 20 bis 300 µm, um ein zu großes Totvolumen zu vermeiden. Über den Auslass (13) erfolgt die Plasmaausgabe.

## Patentansprüche

1. Vorrichtung zur Separierung und Ausgabe von Plasma beinhaltend
- ein Trennelement (1), das ein Trennvlies (3) als einen ersten und ein Transportvlies (2) als einen zweiten Bereich beinhaltet, und
- das Trennelement in der Vorrichtung in der Weise angeordnet ist, dass der erste Bereich für den Benutzer zur Blutaufgabe zugänglich ist,
wobei bei Blutaufgabe auf den ersten Bereich des Trennelementes Plasma in den zweiten Bereich des Trennelementes geleitet wird und korpuskuläre Blutbestandteile im Wesentlichen vollständig im ersten Bereich des Trennelementes zurückgehalten werden, und
- eine Ausgabeeinheit (11, 12), die nach der Plasmaseparation im wesentlichen auf den zweiten Bereich des Trennelementes wirkt, ohne dass eine Einwirkung der Ausgabeeinheit auf den ersten Bereich des Trennelementes erfolgt, sodass das separierte Plasma aus dem zweiten Bereich des Trennelementes freigesetzt wird und über einen Auslass (13) der Vorrichtung abgegeben wird.

2. Vorrichtung gemäß Anspruch 1,
bei dem das Trennelement ein Einmalartikel ist.

3. Vorrichtung gemäß Anspruch 1,
bei dem der erste Bereich des Trennelementes lateral neben dem zweiten Bereich des Trennelementes in der Weise innerhalb der Vorrichtung angeordnet ist, dass die Ausgabeeinheit im wesentlichen senkrecht zu der Ebene, in der sich das Trennelement befindet, auf den zweiten Bereich des Trennelementes wirkt.

4. Vorrichtung gemäß Anspruch 1,
bei der der zweite Bereich des Trennelementes in einer innerhalb der Vorrichtung beweglichen Halterung befestigt ist.

5. Vorrichtung gemäß Anspruch 4,
bei der eine Drehung der Halterung um vorzugsweise 90° möglich ist, wodurch eine Abtrennung des zweiten Bereichs des Trennelementes vom ersten Bereich bewirkt wird.

6. Vorrichtung gemäß Anspruch 1,
bei der der zweiten Bereich des Trennelementes vom ersten Bereich des Trennelementes abgetrennt wird und das Abtrennen sowie das Freisetzen von Plasma aus dem zweiten Bereich in zwei hintereinander folgenden Schritten durch Betätigen einer Auslöseeinheit an der Vorrichtung erfolgt.

7. Vorrichtung gemäß Anspruch 1,
bei der der zweite Bereich des Trennelementes mittels eines Stempels ausgepresst wird.

8. Vorrichtung gemäß Anspruch 1,
bei der das Trennelement streifenförmig ist.

9. System zum Nachweis von Analyten im Blut beinhaltend:
- eine Vorrichtung gemäß einem der Ansprüche 1 bis 8, sowie
- ein Testelement, das bei Aufgabe des separierten Plasmas einen Nachweis eines Analyten im Plasma ermöglicht.

10. System gemäß Anspruch 9,
bei dem der Aufbau des Testelementes in der Weise vereinfacht ist, dass keine Plasmaseparierung durch das Testelement selbst erfolgt.

11. Verfahren zur Plasmaseparierung und -ausgabe beinhaltend
- Aufgabe von Blut auf ein Trennvlies als ersten Bereich eines Trennelements,
- Separierung eines Plasmas von anderen Blutbestandteilen mittels des Trennelementes, wobei die Blutbestandteile im wesentlichen im ersten Bereich des Trennelementes zurückgehalten werden, und das Plasma in ein Transportvlies als zweiten Bereich des Trennelementes weitergeleitet wird,
- anschließend Bearbeitung des zweiten Bereiches des Trennelementes, ohne dass ein Einwirken auf den ersten Bereich des Trennelementes erfolgt, sodass das Plasma aus dem zweiten Bereich des Trennelementes freigesetzt wird, und
- Ausgabe von freigesetztem Plasma über einen Auslass.

12. Verfahren gemäß Anspruch 11,
bei dem der zweite Bereich des Trennelementes vom ersten Bereich des Trennelementes abgetrennt wird.

13. Verfahren gemäß Anspruch 11,
bei dem aus dem zweiten Bereich des Trennelementes das separierte Plasma eluiert wird.

14. Verfahren gemäß Anspruch 11,
bei dem aus dem zweiten Bereich des Trennelementes das separierte Plasma mittels Druck freigesetzt wird.

15. Verfahren gemäß Anspruch 11,
bei dem die Plasmaseparation aufgrund eines Filterungsprozess erfolgt.

16. Verfahren gemäß Anspruch 15,
bei dem durch einen Unterdruck der Filterungsprozess unterstützt wird.

17. Verfahren gemäß Anspruch 11,
das zur Bestimmung von Lipoproteinen mit hoher Dichte verwendet wird.

18. Verfahren gemäß Anspruch 11,
bei dem vorzugsweise das aufgegebene Blutvolumen 30 µl bis 150 µl beträgt.

19. Verfahren gemäß Anspruch 11,
bei dem eine Plasmaabtrennung, -freisetzung und -ausgabe mit einer Vorrichtung gemäß einer der Ansprüche 1 - 9 erfolgt.

## Claims

1. Device for separation and discharging plasma comprising
- a separation element (1) which comprises a separation fleece (3) as a first and a transport fleece (2) as a second zone and
- the separation element is arranged in the device in such a manner that the first zone is accessible for blood application by the user,
wherein when blood is applied to the first zone of the separation element, plasma is passed into the second zone of the separation element and corpuscular blood components are essentially completely retained in the first zone of the separation element and
- a discharge unit (11, 12) which, after plasma separation, acts essentially on the second zone of the separation element without the discharge unit having an effect on the first zone of the separation element so that the separated plasma is released from the second zone of the separation element and is discharged through an outlet (13) of the device.

2. Device according to claim 1,
in which the separation element is a single-use article.

3. Device according to claim 1,
in which the first zone of the separation element is arranged within the device laterally next to the second zone of the separation element in such a manner that the discharge unit acts on the second zone of the separation element essentially perpendicular to the plane in which the separation element is located.

4. Device according to claim 1,
in which the second zone of the separation element is mounted in a movable holder within the device.

5. Device according to claim 4,
in which the holder can be preferably rotated by 90° resulting in a detachment of the second zone of the separation element from the first zone.

6. Device according to claim 1,
in which the second zone of the separation element is detached from the first zone of the separation element and the detachment and release of plasma from the second zone occur in two consecutive steps by actuating a trigger unit on the device.

7. Device according to claim 1,
in which the second zone of the separation element is pressed out by a plunger.

8. Device according to claim 1, in which the separation element is strip-shaped.

9. System for detecting analytes in blood comprising :
- a device according to one of the claims 1 to 8 and
- and a test element which enables detection of an analyte in plasma when the separated plasma is applied.

10. System according to claim 9,
in which the structure of the test element is simplified in such a manner that there is no plasma separation by the test element itself.

11. Method for plasma separation and discharge comprising
- applying blood to a first zone of a separation element,
- separating plasma from other blood components by means of the separation element, the blood components being essentially retained in the first zone of the separation element and the plasma being passed into a transport fleece as the second zone of the separation element,
- subsequently processing the second zone of the separation element without affecting the first zone of the separation element such that plasma is released from the second zone of the separation element and
- discharge of the released plasma through an outlet.

12. Method according to claim 11,
in which the second zone of the separation element is detached from the first zone of the separation element.

13. Method according to claim 11,
in which the separated plasma is eluted from the second zone of the separation element.

14. Method according to claim 11,
in which the separated plasma is released by means of pressure from the second zone of the separation element.

15. Method according to claim 11,
in which plasma is separated on the basis of a filtering process.

16. Method according to claim 15,
in which the filtering process is assisted by negative pressure.

17. Method according to claim 11,
which is used to determine high density lipoproteins.

18. Method according to claim 11,
in which the applied blood volume is preferably 30 µl to 150 µl.

19. Method according to claim 11,
in which plasma separation, release and discharge is carried out with a device according to one of the claims 1 - 9.

## Revendications

1. Dispositif pour la séparation et l'émission de plasma, comportant
- un élément de séparation (1), qui comporte une membrane de séparation (3) comme première zone et une membrane de transport (2) comme deuxième zone, et
- l'élément de séparation est disposé dans le dispositif de telle manière que la première zone est accessible à l'utilisateur pour le dépôt de sang,
où, lors du dépôt de sang sur la première zone de l'élément de séparation, le plasma est guidé dans la deuxième zone de l'élément de séparation et les constituants corpusculaires du sang sont retenus de manière essentiellement complète dans la première zone de l'élément de séparation, et
- une unité d'émission (11/12) qui agit, après la séparation du plasma, essentiellement sur la deuxième zone de l'élément de séparation, sans qu'il ne se produise un effet de l'unité d'émission sur la première zone de l'élément de séparation, de telle manière que le plasma séparé est libéré de la deuxième zone de l'élément de séparation et est évacué du dispositif via une sortie (13).

2. Dispositif selon la revendication 1, dans lequel l'élément de séparation est un objet à usage unique.

3. Dispositif selon la revendication 1, dans lequel la première zone de l'élément de séparation est disposée latéralement à côté de la deuxième zone de l'élément de séparation de telle manière dans le dispositif que l'unité d'émission agit de manière essentiellement perpendiculaire par rapport au plan, dans lequel se trouve l'élément de séparation, sur la deuxième zone de l'élément de séparation.

4. Dispositif selon la revendication 1, dans lequel la deuxième zone de l'élément de séparation est fixée dans un dispositif de fixation qui est mobile dans le dispositif.

5. Dispositif selon la revendication 4, dans lequel une rotation du dispositif de fixation de préférence de 90° est possible, ce qui provoque une séparation de la deuxième zone de l'élément de séparation de la première zone.

6. Dispositif selon la revendication 1, dans lequel la deuxième zone de l'élément de séparation est séparée de la première zone de l'élément de séparation et la séparation et la libération du plasma de la deuxième zone sont réalisées dans deux étapes consécutives en actionnant une unité de déclenchement du dispositif.

7. Dispositif selon la revendication 1, dans lequel la deuxième zone de l'élément de séparation est pressée au moyen d'un tampon.

8. Dispositif selon la revendication 1, dans lequel l'élément de séparation est en forme de bandelette.

9. Système pour la détection d'analytes dans le sang, comprenant :
- un dispositif selon l'une quelconque des revendications 1 à 8, ainsi que
- un élément test, qui permet lors du dépôt du plasma séparé, une détection d'un analyte dans le plasma.

10. Système selon la revendication 9, dans lequel la structure de l'élément test est simplifiée de telle manière qu'il ne se produit pas de séparation du plasma par l'élément test lui-même.

11. Procédé pour la séparation et l'émission de plasma, comprenant
- le dépôt de sang sur une membrane de séparation comme première zone d'un élément de séparation,
- la séparation d'un plasma des autres constituants du sang au moyen de l'élément de séparation, les constituants du sang étant essentiellement retenus dans la première zone de l'élément de séparation et le plasma étant guidé dans une membrane de transport comme deuxième zone de l'élément de séparation,
- le traitement consécutif de la deuxième zone de l'élément de séparation sans qu'il ne se produise un effet sur la première zone de l'élément de séparation, de telle manière que le plasma est libéré par la deuxième zone de l'élément de séparation, et
- l'émission du plasma libéré via une sortie.

12. Procédé selon la revendication 11, dans lequel la deuxième zone de l'élément de séparation est séparée de la première zone de l'élément de séparation.

13. Procédé selon la revendication 11, dans lequel le plasma séparé est élué de la deuxième zone de l'élément de séparation.

14. Procédé selon la revendication 11, dans lequel le plasma séparé est libéré de la deuxième zone de l'élément de séparation au moyen d'une pression.

15. Procédé selon la revendication 11, dans lequel la séparation du plasma se produit sur base d'un processus de filtration.

16. Procédé selon la revendication 15, dans lequel le processus de filtration est soutenu par une dépression.

17. Procédé selon la revendication 11, qui est utilisé pour la détermination de lipoprotéines avec une densité élevée.

18. Procédé selon la revendication 11, dans lequel le volume de sang déposé est de 30 µl à 150 µl.

19. Procédé selon la revendication 11, dans lequel la séparation, la libération et l'émission du plasma sont réalisées au moyen d'un dispositif selon une quelconque des revendications 1- 9.
